# EUROPEAN PATENT SPECIFICATION

(11) **EP 0 563 249 B1**
(45) Date of publication and mention of the grant of the patent: **23.04.1997**
(21) Application number: 92902979.1
(22) Date of filing: 13.12.1991
(51) Int. Cl.: A61K 47/48

(54) **TARGETING OF THERAPEUTIC AGENTS USING POLYSACCHARIDES**
TARGETING VON THERAPEUTISCHEN MITTELN DURCH VERWENDUNG VON POLYSACCHARIDEN
CIBLAGE D'AGENTS THERAPEUTIQUES AU MOYEN DE POLYSACCHARIDES

(30) Priority: 19.12.1990 US 630017
(43) Date of publication of application: 06.10.1993
(73) Proprietor: ADVANCED MAGNETICS, INC., Cambridge, MA 02138-1038 (US)
(72) Inventor: JOSEPHSON, Lee, Arlington, MA 02174 (US); GROMAN, Ernest, V., Brookline, MA 02135 (US); JUNG, Chu, Arlington, MA 02174 (US); LEWIS, Jerome, M., Newton, MA 02161 (US)
(74) Representative: Overbury, Richard Douglas
(86) International application number: US9109368
(87) International publication number: WO9211037

(56) References cited:
- EP-A- 0 281 809
- WO-A-90/01295
- Magnetic Resonance Imaging, vol. 8, no. 5, 1990, Elmsford, NY, US; L. JOSEPHSON et al.: "A funtionalized superparamagnetic iron oxide colloid as a receptor directed MR contrast agent", pages 637-646
- J. American medical Association, vol. 243, no. 17, 2 May 1980, Chicago, IL, US; R.D. HAMSTRA et al.: "Intravenous iron dextran in clinical medicine", pages 1726-1731
- Science, vol. 244, 12 May 1989, Washington, DC, US; A. MUKHOPADHYAY et al.: "Receptor-mediated drug delivery to macrophages in chemotherapy of Leishmaniasis", pages 705-707
- Pharmaceutical Research, vol. 6, no. 2, 1989, Stuttgart, DE; D.K.F. MEIJER et al.: "Covalent and noncovalent protein binding of drugs: Implications for hepatic clearance, storage, and cell-specific drug delivery", pages 105-118
- The Journal of Biological Chemistry, vol. 263, no. 29, 15 October 1988, Baltimore, US; G.Y. WU et al.: "Receptor-mediated gene delivery and expression in vivo", pages 14621-14624
- Carbohydrate Research, vol. 118, 1983, Amsterdam, NL; M.M. PONPIPOM et al.: "Synthesis of 6-(5-cholesten-3beta-yloxy)hexyl 4-0-(6-deoxy-beta-D-galctopyranosyl)-1-thio-beta-D-glucopyranoside and derivatives thereof for in vivo liposome studies", pp. 47-55
- Biochemistry International, vol. 10, no. 3, March 1985, North Ryde, AU; P. DASGUPTA et al.: "Receptor-mediated uptake of asialoganglioside liposomes: Sub-cellular distribution on the liposomal marker in isolated liver cell types", pages 327-338
- Proc. Indian. natn. Sci. Acad., vol. 48, supplement no. 1, 1982, New Delhi, IN; P. GHOSH et al.: "An approach to tissue targeting of drugs and proteins using liposomes", pages 12-19
- Methods in Enzymology, vol. 112, 1985, New York, US; J.L. BODMER et al.: "[23] Carrier potential of glycoproteins", pages 298-306

## Description

### Technical Field of the Invention

The present invention relates to the targeting of a therapeutic agent to a specific population of cells, especially hepatocytes.

### Background of the Invention

Before reviewing the background art, it is useful to define certain terms. A therapeutic agent is one administered with the intent of changing, in a beneficial manner, some physiological function of the recipient. Therapeutic agents include drugs, proteins, hormones, enzymes, nucleic acids, peptides, steroids, growth factors, modulators of enzyme activity, modulators of receptor activity and vitamins. A diagnostic agent is one administered with the intent of illuminating some physiological function, while leaving physiological function unaffected. Diagnostic agents include radioactive isotopes for scintigraphy, electron dense labels for X-ray or computer tomography, and magnetic labels for magnetic resonance imaging.

Targeting is the modification of an agent so that after parenteral administration its uptake by a specific type or population of cells is increased, over that obtained with the unmodified agent.

Receptor mediated endocytosis (RME) is a process whereby molecules in the extracellular space bind to specific receptors on the cell surface and are internalized. Through the process known as RME, molecules injected into the vascular compartment are cleared (removed) from plasma.

Uptake by RME exhibits three general properties characteristic of ligand-receptor interactions generally: structural specificity, saturability and competition. Structural specificity is observed when a receptor can distinguish between closely related structures and only molecules with structures meeting the binding requirements of the receptor binding site are internalized. Often the receptors involved in RME are discovered by their ability to internalize or clear glycoproteins from circulation. Saturability is observed when the rate of an agent internalized via RME decreases with increasing concentrations of that agent. This results because, at high concentrations, the receptor approaches full occupancy or becomes saturated with ligand.

Competition is observed when the rate of internalization of an agent can be reduced by the presence of additional agents bearing a structural resemblance to the first agent. The additional agents compete for receptor binding sites and decrease the rate of internalization of the first agent. Saturability results when high concentrations of a single ligand compete for a limited number of receptor sites. Competition results when chemically different ligands bind to a limited number of receptor sites.

The uptake of substances by RME is a feature of normal, healthy cells. RME transport systems can be found on normal macrophages, hepatocytes, fibroblasts and reticulocytes. RME enables cells to internalize a variety of macromolecules in plasma, such as asialoglycoproteins, low density lipoproteins, transferrin and insulin. See Table 1 of Wileman et al., 232 Biochem. J. (1985) pp. 1-14 for a list of cells performing RME, which also contains a general review of RME. See also Table I of Menz, E.T., PCT WO 90/01295, filed August 3, 1989. Conversion of normal cells to tumor cells (transformation) may be associated with an increase or decrease in the activity of receptors performing RME. In some cases, such as the RME performed by the asialoglycoprotein receptor of hepatocytes, transformation to cancerous hepatoma cells is associated with receptor loss. Stockert et al., 40 Cancer Res. (1980) pp. 3632-3634. In many cases, like the antibody based targeting of drugs to tumor antigens, the antigens are increased on tumor cells and decreased on normal cells.

Polysaccharides like arabinogalactan, which interact with receptors involved in RME, are referred to as RME-type polysaccharides. Many common polysaccharides such as dextrans, dextrins, celluloses, hydroxyethylstarches, heparins, starches, dextran sulfates, carboxylmethylated dextran and carboxymethyl cellulose do not interact with receptors involved in RME; they are referred to as non-RME polysaccharides.

With these definitions in hand, the relevant background art will be discussed. Non-RME type polysaccharides have been used in the synthesis of a variety of materials used as diagnostic or therapeutic agents. Jacobsen, T., EPO O 186 947 B1; Schroder, USP 4,501,726; Ranney, D.F., PCT WO 90/03190, filed September 29, 1989; Groman, USP 4,827,945; Groman, USP 4,770,183. Ranney discloses the delivery of diagnostic agents (metal ions as magnetic resonance (MR) contrast agents) using a polymeric carrier which is directed to tumor cells. Ranney suggests, without detailed examples, that other therapeutic complexes may also be delivered using this method, for chemotherapeutic impact or to provide sensitization or augmentation for radiation treatment (Ranney, D.F., PCT WO 90/03190, filed September 29, 1989, p. 51). It is known that the RME-type polysaccharide arabinogalactan can be used to target certain diagnostic agents, particularly superparamagnetic iron oxide. Menz, E.T., PCT WO 90/01295, filed August 3, 1988.

Therapeutic agents, on the other hand, have been typically targeted by liposomes and glycoproteins. Normally after injection, liposomes are recognized as particulate matter and are subject to phagocytosis, which results in their concentration in the tissues of the reticuloendothelial system (RES). Materials within liposomes are then concentrated in tissues such as the liver, spleen and bone which comprise the RES. Surface-modified liposomes have been synthesized and can be cleared by RME, but the surface modification consisted of a coating of proteins or glycoproteins. Ranade, V.V., 29 J. Clin. Pharmacol. (1989) pp. 685-694; Dragsten et al., 926 Biochem. Biophys. Acta (1987) pp. 270-279.

Colloids and particles of differing sizes and compositions are recognized by the RES. For example, Imferon, a dextran coated colloidal ferric oxyhydroxide used for the treatment of anemia, is slowly cleared from the blood by the phagocytic activity of the macrophages of the RES. (The word "Imferon" is a trade mark) Henderson et al., 34 Blood (1969) pp. 357-375. Radioactive diagnostic agents such as the technicium sulfur colloids and many types of magnetic particles used as MR contrast agents are also cleared by the RES. For a discussion see Josephson et al., 8 Mag. Res. Imag. (1990) pp. 637-646.

Glycoproteins internalized by RME have been used to target therapeutic agents. For a review of targeting strategies see Table II of Meijer et al., 6 Pharm. Res. (1989) pp. 105-118.

### Summary of the Invention

The present invention provides a complex for use in targeting a therapeutic agent to a specific population of cells. Targeting is accomplished by forming a covalently bound complex between a therapeutic agent and a polysaccharide capable of interacting with receptors performing receptor mediated endocytosis (RME). The resulting complex is then internalized into the specific population of cells by receptor mediated endocytosis. The invention enables the concentration of therapeutic agents to be increased in tissues where they have beneficial actions and decreased in tissues where they have unwanted, toxic effects.

### Detailed Description of the Specific Embodiments

### General:

The invention provides a complex for targeting a therapeutic agent into a specific population of cells. Targeting increases the concentration of the therapeutic agent in cells where the agent exerts some beneficial action and reduces its concentration in other cells where unwanted, toxic effects are being produced. Many therapeutic agents produce toxic effects, not upon the cells where the agent has a beneficial action, but on cells other than those responsible for the beneficial action.

By targeting therapeutic agents towards certain cells, and away from other cells, the invention provides a way of improving the safety and efficacy of previously developed therapeutic agents. For example, a therapeutic agent intended to modify the metabolism of the hepatocytes of the liver, might exhibit toxic effects to bone marrow cells. Since bone marrow function is essential for life, toxic effects on marrow limit the dose of the agent that can be given. If the agent were targeted to hepatocytes by attachment to the arabinogalactan, the concentration to bone marrow would be reduced. The potency of the agent would be improved, because the fraction of the therapeutic agent which normally goes to bone marrow is now directed to the liver. Bone marrow related side effects would be eliminated.

### Distinguishing the RME-type Polysaccharides Used by the Invention:

With the current invention, a therapeutic agent is attached to an RME-type polysaccharide and the resulting complex is targeted into specific types of cells through the action of cell surface receptors. Only certain polysaccharides may be used in the invention and these are termed RME-type polysaccharides. RME-type polysaccharides differ from common, non-RME polysaccharides, e.g., dextrans, dextrins, celluloses, hydroxyethylstarches, heparins, starches, dextran sulfates, carboxylmethylated dextran and carboxymethyl cellulose. Non-RME polysaccharides are used in diverse applications such as drug delivery, drug formulation, as food additives and in plasma volume expansion. RME-type polysaccharides include arabinogalactan and mannan, and may be used, according to the invention, to deliver therapeutic agents directly to hepatocytes and macrophages respectively. References, such as Ranney, described above, concerning the delivery of certain therapeutic agents using polysaccharides, do not disclose or concern themselves with the use of RME-type polysaccharides.

Below, we refer to the complex of the invention as the RME-type polysaccharide-therapeutic agent complex. The complex between the RME-type polysaccharide and the therapeutic agent involves the covalent attachment of the therapeutic agent to the RME-type polysaccharide (Examples 1 and 2).

Chemical modifications of non-RME polysaccharides have been achieved, including carboxymethylation, succinylation, hydroxyethylation and sulfation. Generally, such chemical modification of common polysaccharides does not confer the ability to bind to a receptor and undergo RME.

However, non-RME polysaccharides can, in some instances, be modified by the attachment of substituent groups that are recognized by receptors performing RME, and such modifications confer the property of RME on non-RME polysaccharides. For example, a galactose residue can be attached to the non-RME polysaccharide dextran; the galactose of the resulting polysaccharide will be recognized by the asialoglycoprotein receptor and undergo RME. By attachment of galactose, the dextran is converted into an RME-type polysaccharide. Similarly, a mannose group can be attached to dextran and the resulting polysaccharide will be recognized by the mannose receptor of phagocytes.

A second modification of RME-type polysaccharides involves partial digestion to produce lower molecular weight polysaccharides. This can be accomplished by controlled hydrolysis with acid and fractionation to obtain RME-type polysaccharides in the desired size class. The polysaccharides of the invention, before degradation or modification, have molecular weights greater than about 1,000 daltons.

For a polysaccharide to be designated an RME-type polysaccharide, its binding to a receptor performing RME must be demonstrated. One type of demonstration involves the ability of an RME-type polysaccharide to block the clearance of a glycoprotein known to be cleared by RME. For example, the interaction of arabinogalactan with the asialoglycoprotein receptor was demonstrated by its ability to block the clearance of a radioactive sample of the asialoglycoprotein, asialofetuin. Injection of 500 mg/kg of arabinogalactan blocks the clearance of ¹²⁵I-asialofetuin in rats. (See Table 1 of Josephson et al., 8 Mag. Res. Imag. (1990) pp. 637-646.) As a result of this experiment as well as others, it can be concluded that arabinogalactan is recognized by the asialoglycoprotein receptor of hepatocytes. Consequently, arabinogalactan is an RME-type polysaccharide.

Similarly, mannan blocks the clearance of radioactive glycoprotein, RNase B. Brown et al., 188 Arch. Biochem. Biophys. (1978) pp. 418-428. Arabinogalactan and mannan are discussed briefly below. In addition to the polysaccharides discussed explicitly herein, other RME-type polysaccharides may be formed as modification or degradation products of the polysaccharides discussed.

A simple test for whether a polysaccharide-therapeutic agent complex is of the type covered by the invention is afforded by the ability of various substances to slow the elimination of the complex from blood (clearance). The complexes of the invention are cleared by RME, and their clearance is blocked by substances cleared by the same receptor.

The clearance of the RME-type polysaccharide-therapeutic agent complexes of the invention is unaffected by the injections of substantial concentrations of non-RME type polysaccharides, e.g., dextran and hydroxyethyl starch. The clearance of the RME-polysaccharide-therapeutic agents of the invention is also unaffected by the injection of substantial concentrations of particles, colloids or liposomes cleared by the phagocytic cells of the RES.

### Advantages of Polysaccharides as Carriers for the Delivery of Therapeutic Agents:

An advantage of using polysaccharides instead of proteins for the delivery of therapeutic agents is that polysaccharides do not denature readily at high temperature, extremes of pH or in organic solvents. Because of the stability of polysaccharides, covalent linkages between therapeutic agents and polysaccharides can be achieved in organic solvents. This is a considerable advantage since some therapeutic agents have low water solubility. A related advantage of polysaccharides working in nonaqueous media is that water unstable linkages like esters can be created between the therapeutic agent and the polysaccharide. An example of such chemistry is provided in Example 2.

Another advantage of polysaccharides is that they can be obtained from microbiological or plant sources. Glycoproteins from human or animal sources may contain pathogens whose absence is costly to assure. Polysaccharides from microbiological or plant sources can be selected for use in the invention which are of very low toxicity and immunogenicity. Plant or microbiological sources can provide crude polysaccharide preparations on a large scale, in a reliable manner and at a reasonable price. Two classes of carbohydrates which can be utilized in the invention are the arabinogalactans and the mannans.

### Arabinogalactans:

Arabinogalactans are a class of polysaccharides that may be obtained from the cell walls of many species of trees and plants. A common source of arabinogalactan is the American western larch (Larix occidentalis). Arabinogalactan from this source is used as a binder, emulsifier or stabilizer in foods. It consists of a galactose backbone with branch chains of arabinoses and galactose. Generally, the ratio of galactose to arabinose is between 5:1 and 10:1. The molecular weight can be between 10 to 100 kilodaltons. Glickman, ed., "Food Hydrocolloids," CRC Press (1982) pp. 5, 33.

Best results are obtained when a purified arabinogalactan is used. Commercially available arabinogalactan can be further purified by ultrafiltration to remove impurities greater than 100,000 daltons and smaller than 10,000 daltons. Arabinogalactan purified by this method is used in the examples of the patent.

Arabinogalactans bind to the asialoglycoprotein receptor of hepatocytes. This receptor performs RME on a variety of substances. Harford et al., Vol. IV "The Glycoconjugates," M.I. Horowitz, ed., Academic Press (1982) pp. 27-55. Therapeutic agents attached to arabinogalactan will be targeted to hepatocytes.

### Mannans:

Mannans are a class of polysaccharides that can be obtained from the cell walls of yeasts. They are predominantly α-D-mannopyrans with a variety of linear and branched chain structures. Gorin et al., Vol. 2 "The Polysaccharides," G.O. Aspinall, ed., Academic Press (1983) pp. 376-380.

Mannans bind to the mannose receptor found on the macrophages of the RES. Therapeutic agents attached to mannan will be targeted to macrophages.

### Therapeutic Agents Targeted by the Invention:

Utilizing the methods of the invention, a wide variety of therapeutic agents may be targeted to a population of cells. Examples of such therapeutic agents are listed in Table 1. Some of the agents in Table 1 may be targeted to hepatocytes, such as antiviral agents for the treatment of hepatitis. Iron may be targeted to hepatocytes to remedy nutritional imbalance, i.e., iron deficiency anemia. When genetic defects are expressed in the liver, such as the deficiency of a hepatic enzyme, DNA may be targeted to the liver to alter the genetic defects. The invention may be used to target therapeutic agents that have been targeted by other techniques. Other summaries of therapeutic agents whose targeting has been attempted are available. See Table II of Meijer et al., 6 Pharm. Res. (1989) pp. 105-118 and Ranade, 29 J. Clin. Pharmacol. (1989) pp. 685-694.

**Table 1**

| **Applications and Agents Targeted by the Invention** | | |
|---|---|---|
| Agent | Application | Reference |
| Iron | treatment of anemia | Hamstra et al, 243 JAMA 243; 1726-1731 (1980) |
| Ara A-phosphate | hepatitis treatment | Bodmer et al., 112 Methods in Enzymology (1985) pp. 298-306 |
| Triflour-Thymidine | hepatitis treatment | above |
| DNA | genetic defect reversal | Wu et al., 263 J. Biol. Chem. (1988) pp. 14621-14624 |
| Methotrexate | treatment of leishmaniasis | Mukhopadhyay et al., 244 Sci. (1989) pp. 705-707 |

The targeting of antiviral agents into hepatocytes of an individual chronically infected with the hepatitis B virus, is an application of the invention where antiviral agents would be the therapeutic agents targeted. The targeting of an antiviral agent to the infected cell population (hepatocytes), and away from bone marrow, may result in more effective treatment with the drug. Antiviral agents may be attached to arabinogalactan, and injected intravenously, to achieve a high concentration in the hepatocytes. The targeting of nutritionally required substances such as iron may be targeted by the invention. This difference in pharmacokinetics and biodistribution may result in the iron of the invention being a safer therapeutic agent than iron oxide dextran.

Vitamins may also be targeted by the invention. Example 1 shows the preparation of a folic acid arabinogalactan conjugate, which would target the vitamin folic acid to hepatocytes via RME. Folic acid is chemically similar to the drug methotrexate, which can be coupled to arabinogalactan by minor modifications of the procedure shown for folic acid.

Hormones such as steroids may be delivered directly to a specific population of cells utilizing the methods of the invention. Steroids have powerful biological activities which are exerted after the steroid binds to a receptor present on the cells. Martin, C.R., "Textbook of Endocrine Physiology," Williams & Wilkins (1976) p. 21. The targeting of steroids to cells is a widely useful application of the invention. One application of targeting hormones involves targeting glucocorticoid steroids into cells. Example 2 presents a synthesis of an arabinogalactan-prednisone conjugate which may serve to target the steroid prednisone via RME into hepatocytes. Steroids could be targeted by attachment to mannan, and targeted into appropriate cells by the mannose receptor present on cells of the RES.

### Examples

### Example 1:

Folic acid is a vitamin which has been coupled to a polysaccharide undergoing RME called arabinogalactan as described below. The drug methotrexate is a folic acid antagonist and anticancer drug. Methotrexate may be attached to polysaccharides undergoing RME and used in drug delivery applications, by modifying the folic acid coupling chemistry shown below.

Folic acid dihydrate (6.0 mg, 13 µmol) was suspended in H₂O (1 mL). NaOH (0.10 N, 7 drops) was added until the white solid folic acid was almost completely dissolved. Purified arabinogalactan (23,000 daltons, 35.3 mg, 1.53 µmol) was added, followed by 1-(3-dimethylaminopropyl)-3-ethylcarbodiimide (51.2 mg, 286 µmol). After stirring for 2.5 hours at room temperature, the reaction mixture was analyzed by HPLC on a Sephadex G-25 column (9.5 X 300 mm) using an eluent of 0.05% NaN₃ (0.33 mL/min). (The word Sephadex is a trade mark) Detection of free and coupled folic acid was accomplished by using a UV detector, set at 280 nm (for folic acid, UVₘₐₓ = 283 nm, log = 4.40). The chromatogram showed a peak with a retention time of 16.8 minutes due to folate conjugated to arabinogalactan. Free folic acid appeared at 35 minutes. These assignment were obtained from chromatographing arabinogalactan and folic acid. Purified arabinogalactan required a refractive index detector as it does not absorb at 280 nm. Based on UV detection, 37% of the folic acid was coupled to arabinogalactan. Based on no loss of arabinogalactan and 37% of the folate conjugated, a folate/arabinogalactan ratio of 3:1 was obtained.

### Example 2:

Steroids are a class of drugs which can be delivered to cells by attaching them to polysaccharides that undergo RME. A variety of steroids may be coupled to such polysaccharides following analogous chemistry to that given below. The general steps are (i) preparation of a polysaccharide conjugate providing carboxyl groups by reaction with DTPA, and (ii) attachment of the steroid through the carboxyl group of the DTPA-polysaccharide.

### Preparation of Arabinogalactan-DTPA:

Purified arabinogalactan (23,000 daltons, 0.50 g, 21.7 µmol) and diethylenetriaminepentaacetic acid (DTPA) dianhydride (0.102 g, 285 µmol) were dissolved in DMSO (20 mL) at 60°C. After one hour, the clear solution was cooled to room temperature. Upon addition of H₂O (10 mL), a white precipitate formed. The mixture was filtered on an Amicon YM 5 ultrafiltration membrane (5,000 dalton cutoff), and washed with H₂O (4 X 30 mL). The product remaining on the membrane was dissolved in H₂O (10 mL), frozen and lyophilized. Yield of white powder: 0.44 g. The nominal DTPA/arabinogalactan ration was 13:1, assuming attachment of all DTPA added (nominal formula weight: 28,000 daltons).

### Coupling 6α-Methylprednisolone to arabinogalactan-DTPA:

Arabinogalactan-DTPA (107.5 mg, 3.8 µmole) and 6α-methylprednisolone (64.5 mg, 172 µmol) were dissolved in DMSO (15 mL) at 60°C. 1-(3-dimethylaminopropyl)-3-ethylcarbodiimide (259 mg, 1.45 mmol) was added and the reaction mixture allowed to stir at 60°C for one hour. HPLC analysis (Sephadex G-10 column of 9.5 X 300 mm with an eluent of 0.05% NaN₃, 0.50 mL/min, 280 nm UV detector) of the reaction mixture showed only a single peak at 10.5 minutes retention time corresponding to the mobility of the arabinogalactan-DTPA conjugate. No peak from 6α-methylprednisolone at 19.5 minutes was observed, indicating complete attachment (by esterification) of the steroid to the arabinogalactan-DTPA conjugate. After addition of H₂O (10 mL), the reaction mixture was ultrafiltered using an Amicon YM 3 (3,000 dalton cutoff) and washed with H₂) (3 X 30 mL). The filtrate contained unreacted steroid, carbodiimide, traces of DTPA and other low molecular weight materials. HPLC analysis of the filtrate confirmed the absence of free steroid. H₂O (10 mL) was added to the retentate and the product lyophilized. Yield of off-white powder: 0.10 g.

## Claims (Claims for the following Contracting State(s): AT, BE, CH, DE, DK, FR, GB, IT, LI, LU, MC, NL, SE)

1. A complex for use in targeting a therapeutic agent to a specific population of cells, which complex comprises
a carrier capable of binding to an RME receptor and selected from the group consisting of a polysaccharide and a modification thereof, and
the therapeutic agent covalently bonded to the carrier such that the therapeutic agent may be targeted to the RME receptor on a cellular target and internalised therewith.

2. A complex according to claim 1, wherein the carrier is selected from the group consisting of arabinogalactan, mannan, fucoidan and a degradation product thereof.

3. A complex according to claim 1 or 2 wherein the carrier is a polysaccharide modified by a functional residue to form a derivative.

4. A complex according to claim 1, 2 or 3, wherein the therapeutic agent is a nutritionally required substance.

5. A complex according to claim 1, 2 or 3, wherein the therapeutic agent is an antiviral agent.

6. A complex according to claim 1, 2 or 3, wherein the therapeutic agent is a hormonal agent.

7. A complex according to claim 1, 2 or 3, wherein the therapeutic agent is one of a gene, an enzyme, a vitamin, methotrexate, folic acid, 6a-methylprednisone or trifluorthymidine.

8. A complex according to claim 5, wherein the therapeutic agent is ara-A-phosphate.

9. A complex according to claim 7, wherein the therapeutic agent is DNA.

10. The use of a complex for the preparation of a medicament wherein the complex comprises a polysaccharide carrier covalently bonded to a therapeutic agent wherein the therapeutic agent is selected from the group of agents consisting of a nutritional agent, an antiviral agent, and a hormone for delivery of the agent to an RME receptor on a specific population of cells and internalisation therewith.

## Claims (Claims for the following Contracting State(s): ES, GR)

1. Use of a complex for the preparation of a medicament for targeting a therapeutic agent to a specific population of cells, wherein the complex comprises
a carrier capable of binding to an RME receptor and selected from the group consisting of a polysaccharide and a modification thereof, and
the therapeutic agent covalently bonded to the carrier such that the therapeutic agent may be targeted to the RME receptor on a cellular target and internalised therewith.

2. Use of a complex according to claim 1, wherein the carrier is selected from the group consisting of arabinogalactan, mannan, fucoidan and a degradation product thereof.

3. Use of a complex according to claim 1 or 2 wherein the carrier is a polysaccharide modified by a functional residue to form a derivative.

4. Use of a complex according to claim 1, 2 or 3, wherein the therapeutic agent is a nutritionally required substance.

5. Use of a complex according to claim 1, 2 or 3, wherein the therapeutic agent is an antiviral agent.

6. Use of a complex according to claim 1, 2 or 3, wherein the therapeutic agent is a hormonal agent.

7. Use of a complex according to claim 1, 2 or 3, wherein the therapeutic agent is one of a gene, an enzyme, a vitamin, methotrexate, folic acid, 6a-methylprednisone or trifluorthymidine.

8. Use of a complex according to claim 5, wherein the therapeutic agent is ara-A-phosphate.

9. Use of a complex according to claim 7, wherein the therapeutic agent is DNA.

10. A method of preparing a complex for use in the preparation of a medicament for targeting a therapeutic agent to a specific population of cells, the method comprising attaching a therapeutic agent to an RME-type polysaccharide by means of a covalent bond.

## Patentansprüche (Patentansprüche für folgende(n) Vertragsstaat(en): AT, BE, CH, DE, DK, FR, GB, IT, LI, LU, MC, NL, SE)

1. Komplex zum Targeting eines therapeutischen Mittels auf eine bestimmte Population von Zellen, umfassend einen Träger, der an einen RME-Rezeptor binden kann und ausgewählt ist aus der Gruppe Polysaccharid und dessen Modifikationen, wobei das therapeutische Mittel kovalent an den Träger gebunden ist, so daß das therapeutische Mittel an den RME-Rezeptor einer Zielzelle gelenkt und dort internalisiert wird.

2. Komplex nach Anspruch 1, wobei der Träger ausgewählt ist aus der Gruppe Arabinogalaktan, Mannan, Fukoidan und deren Abbauprodukten.

3. Komplex nach Anspruch 1 oder 2, wobei der Träger ein Polysaccharid ist, modifiziert durch einen funktionellen Rest, so daß ein Derivat vorliegt.

4. Komplex nach Anspruch 1, 2 oder 3, wobei das therapeutische Mittel eine für die Ernährung wichtige Substanz ist.

5. Komplex nach Anspruch 1, 2 oder 3, wobei das therapeutische Mittel ein antivirales Mittel ist.

6. Komplex nach Anspruch 1, 2 oder 3, wobei das therapeutische Mittel ein Hormonmittel ist.

7. Komplex nach Anspruch 1, 2 oder 3, wobei das therapeutische Mittel ein Gen, ein Enzym, ein Vitamin, Methotrexat, Folsäure, 6α-Methylprednison oder Trifluorthymidin ist.

8. Komplex nach Anspruch 5, wobei das therapeutische Mittel Ara-A-Phoshat ist.

9. Komplex nach Anspruch 7, wobei das therapeutische Mittel DNA ist.

10. Verwendung des Komplexes zur Herstellung eines Medikaments, wobei der Komplex einen Polysaccharidträger umfaßt, der kovalent an ein therapeutisches Mittel gebunden ist, wobei das therapeutische Mittel ausgewählt ist aus der Gruppe: Nahrungsmittel, antivirale Mittel, Hormone zur Abgabe des Mittels an den RME-Rezeptor einer bestimmten Population von Zellen und zur Internalisation mit den Zellen.

## Patentansprüche (Patentansprüche für folgende(n) Vertragsstaat(en): ES, GR)

1. Verwendung eines Komplexes zur Herstellung eines Medikamentes zum Targeting eines therapeutischen Mittels auf eine bestimmte Population von Zellen, wobei der Komplex einen Träger umfaßt, der an einen RME-Rezeptor binden kann und ausgewählt ist aus der Gruppe Polysaccharid und dessen Modifikationen, wobei das therapeutische Mittel kovalent an den Träger gebunden ist, so daß das therapeutische Mittel an den RME-Rezeptor einer Zielzelle gelenkt und dort internalisiert wird.

2. Verwendung eines Komplexes nach Anspruch 1, wobei der Träger ausgewählt ist aus der Gruppe Arabinogalaktan, Mannan, Fukoidan und deren Abbauprodukten.

3. Verwendung eines Komplexes nach Anspruch 1 oder 2, wobei der Träger ein Polysaccharid ist, modifiziert durch einen funktionellen Rest, so daß ein Derivat vorliegt.

4. Verwendung eines Komplexes nach Anspruch 1, 2 oder 3, wobei das therapeutische Mittel eine für die Ernährung wichtige Substanz ist.

5. Verwendung eines Komplexes nach Anspruch 1, 2 oder 3, wobei das therapeutische Mittel ein antivirales Mittel ist.

6. Verwendung eines Komplexes nach Anspruch 1, 2 oder 3, wobei das therapeutische Mittel ein Hormonmittel ist.

7. Verwendung eines Komplexes nach Anspruch 1, 2 oder 3, wobei das therapeutische Mittel ein Gen, ein Enzym, ein Vitamin, Methotrexat, Folsäure, 6α-Methylprednison oder Trifluorthymidin ist.

8. Verwendung eines Komplexes nach Anspruch 5, wobei das therapeutische Mittel ara-A-Phoshat ist.

9. Verwendung eines Komplexes nach Anspruch 7, wobei das therapeutische Mittel DNA ist.

10. Verfahren zur Herstellung eines Komplexes zur Verwendung in der Herstellung eines Medikaments zum Targeting eines therapeutischen Mittels auf eine bestimmte Population von Zellen, umfassend das Anbringen eines therapeuischen Mittels an ein Polysaccharid vom RME-Typ durch kovalente Bindung.

## Revendications (Revendications pour l'(les) Etat(s) contractant(s) suivant(s): AT, BE, CH, DE, DK, FR, GB, IT, LI, LU, MC, NL, SE)

1. Complexe destiné à être utilisé pour le ciblage d'un agent thérapeutique vers une population de cellules spécifique, lequel complexe comprend
un porteur pouvant se lier à un récepteur RME et choisi dans le groupe se composant d'un polysaccharide et d'une modification de celui-ci, et
l'agent thérapeutique lié de manière covalente au porteur de telle sorte que l'agent thérapeutique puisse être ciblé vers le récepteur RME sur une cible cellulaire et internalisé avec elle.

2. Complexe selon la revendication 1, dans lequel le porteur est choisi dans le groupe se composant de l'arabinogalactane, du mannane, du fucoïdane et d'un produit de dégradation de ceux-ci.

3. Complexe selon la revendication 1 ou 2, dans lequel le porteur est un polysaccharide modifié par un résidu fonctionnel pour former un dérivé.

4. Complexe selon la revendication 1, 2 ou 3, dans lequel l'agent thérapeutique est une substance nécessaire nutritionnellement.

5. Complexe selon la revendication 1, 2 ou 3, dans lequel l'agent thérapeutique est un agent antiviral.

6. Complexe selon la revendication 1, 2 ou 3, dans lequel l'agent thérapeutique est un agent hormonal.

7. Complexe selon la revendication 1, 2 ou 3, dans lequel l'agent thérapeutique est un gène, une enzyme, une vitamine, du méthotréxate, de l'acide folique, de la 6a-méthylprednisone ou de la trifluorthymidine.

8. Complexe selon la revendication 5, dans lequel l'agent thérapeutique est de l'ara-A-phosphate.

9. Complexe selon la revendication 7, dans lequel l'agent thérapeutique est de l'ADN.

10. Utilisation d'un complexe pour la préparation d'un médicament dans laquelle le complexe comprend un porteur de polysaccharide lié de manière covalente à un agent thérapeutique, l'agent thérapeutique étant choisi dans le groupe d'agents se composant d'un agent nutritionnel, d'un agent antiviral, et d'une hormone pour la libération de l'agent vers un récepteur RME dans une population spécifique de cellules et pour l'internalisation avec elle.

## Revendications (Revendications pour l'(les) Etat(s) contractant(s) suivant(s): ES, GR)

1. Utilisation d'un complexe pour la préparation d'un médicament pour le ciblage d'un agent thérapeutique vers une population de cellules spécifique, dans laquelle le complexe comprend
un porteur pouvant se lier à un récepteur RME et choisi dans le groupe se composant d'un polysaccharide et d'une modification de celui-ci, et
l'agent thérapeutique lié de manière covalente au porteur de telle sorte que l'agent thérapeutique puisse être ciblé vers le récepteur RME sur une cible cellulaire et internalisé avec elle.

2. Utilisation d'un complexe selon la revendication 1, dans laquelle le porteur est choisi dans le groupe se composant de l'arabinogalactane, du mannane, du fucoïdane et d'un produit de dégradation de ceux-ci.

3. Utilisation d'un complexe selon la revendication 1 ou 2, dans laquelle le porteur est un polysaccharide modifié par un résidu fonctionnel pour former un dérivé.

4. Utilisation d'un complexe selon la revendication 1, 2 ou 3, dans laquelle l'agent thérapeutique est une substance nécessaire nutritionnellement.

5. Utilisation d'un complexe selon la revendication 1, 2 ou 3, dans laquelle l'agent thérapeutique est un agent antiviral.

6. Utilisation d'un complexe selon la revendication 1, 2 ou 3, dans laquelle l'agent thérapeutique est un agent hormonal.

7. Utilisation d'un complexe selon la revendication 1, 2 ou 3, dans laquelle l'agent thérapeutique est un gène, une enzyme, une vitamine, du méthotréxate, de l'acide folique, de la 6a-méthylprednisone ou de la trifluorthymidine.

8. Utilisation d'un complexe selon la revendication 5, dans laquelle l'agent thérapeutique est de l'ara-A-phosphate.

9. Utilisation d'un complexe selon la revendication 7, dans laquelle l'agent thérapeutique est de l'ADN.

10. Méthode de préparation d'un complexe pour une utilisation dans la préparation d'un médicament pour le ciblage d'un agent thérapeutique vers une population de cellules spécifique, la méthode comprenant l'attachage d'un agent thérapeutique à un polysaccharide du type RME au moyen d'une liaison covalente.
